# EUROPÄISCHE PATENTSCHRIFT

(11) **EP 0 483 062 B1**
(45) Veröffentlichungstag und Bekanntmachung des Hinweises auf die Patenterteilung: **18.09.1996**
(21) Anmeldenummer: 91810795.4
(22) Anmeldetag: 15.10.1991
(51) Int. Cl.: C07D 213/61, A01N 43/40, C07D 213/40, C07D 277/32, A01N 43/78

(54) **Verfahren zur Herstellung von Nitroguanidinderivaten**
Process for the preparation of nitroguanidine derivatives
Procédé pour la préparation de dérivés de la nitroguanidine

(30) Priorität: 24.10.1990 CH 3395/90
(43) Veröffentlichungstag der Anmeldung: 29.04.1992
(73) Patentinhaber: CIBA-GEIGY AG, 4002 Basel (CH)
(72) Erfinder: Maienfisch, Peter, Dr., CH-4118 Rodersdorf (CH); Kristiansen, Odd, Dr., CH-4313 Möhlin (CH); Gsell, Laurenz, Dr., CH-4056 Basel (CH)

(56) Entgegenhaltungen:
- EP-A- 0 375 907
- EP-A- 0 386 565
- EP-A- 0 428 941
- EP-A- 0 483 055
- WO-A-91/01978
- JP-A-03 291 267
- US-A- 4 937 340
- CHEMICAL ABSTRACTS, vol. 116, no. 23, Columbus, Ohio, US; abstract no. 235455E, F. WU ET AL: 'Preparation of substituted nitroguanidines as insecticides' Seite 809 ;
- Journal of Organic Chemistry, vol. 38, p. 2931-9 (1975)

## Beschreibung

Die vorliegende Erfindung betrifft ein neuartiges Verfahren zur Herstellung von substituierten 2-Nitroguanidinderivaten.

Es ist bereits bekannt, dass zur Herstellung von 1,3-disubstituierten 2-Nitroguanidinen, in monosubstituierte 2-Nitroguanidine ein weiterer Substituent (z.B. durch Alkylierung) eingeführt werden kann (vgl. z.B. die EP-Patentanmeldungen 0.375.907, 0.376.279 und 0.383.091). Aufgrund des Vorliegens von drei reaktionsfähigen Wasserstoffatomen in den bei diesen Umsetzungen als Ausgangsmaterial verwendeten monosubstituierten 2-Nitroguanidinen verlaufen die bisher vorgeschlagenen Substitutionsreaktionen dieser Art oftmals unselektive und führen zu unerwünschten Substitutionsprodukten. In den erwähnten EP-Patentanmeldungen wird die Herstellung von 1,3-disubstituierten 2-Nitroguanidinen durch Umsetzung von monosubstituierten Nitroisothioharnstoffen mit primären Aminen unter Mercaptan-Abspaltung beschrieben. Diese bei den bekannten Verfahren als Ausgangsverbindungen vorgeschlagenen Alkylthio-Abgangsgruppen enthaltenden Nitroisothioharnstoffverbindungen sind aber nur schwer zugänglich. US-P-4'937'340 lehrt, daß 2-Nitroiminohexahydro-1,3,5 triazine hydrolyse-bestandig sind, weshalb eine Synthese der 1,3-disubstituierten 2-Nitroguanidine via die leicht Zugänglichen 1,3-disubstituierten 2-nitroiminohexahydro-1,3,5-triazine als ausgeschlossen erscheint.

Ziel der vorliegenden Erfindung ist ein verbessertes Verfahren zur Herstellung von 1-monosubstituierten und 1,3-disubstituierten 2-Nitroguanidinen aus leicht erhältlichen Ausgangsverbindungen, welche eine gezielte 1,3-Disubstitution ohne Entstehung grösserer Mengen unerwünschter Nebenprodukte gestattet.

In Anbetracht des Standes der Technik wurde einer überraschenderweise ein Verfahren zur Herstellung einer Verbindung der Formel 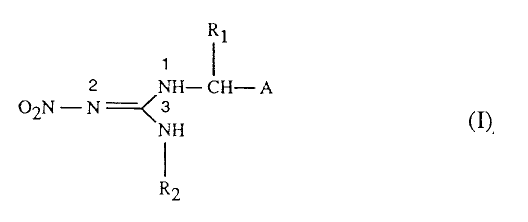 worin
- R₁: Wasserstoff oder C₁-C₄-Alkyl,
- R₂: Wasserstoff, C₁-C₆-Alkyl, C₃-C₆-Cycloalkyl oder einen Rest -CH₂B;
- A: einen unsubstituierten oder, je nach Substitutionsmöglichkeiten des Ringsystems, ein- bis vierfach substituierten Pyridyl-, 1-Oxidopyridinio- oder Thiazolyl-Rest, der ein bis zwei Substituenten aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃-Halogenalkenyl und C₂-C₃-Halogenalkinyl mit 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyan und Nitro und ein bis vier Substituenten aus der Gruppe C₁-C₃-Alkyl, C₁-C₃-Alkoxy und Halogen enthalten kann; und
- B: Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, durch ein bis zwei Substituenten aus der Gruppe C₁-C₃-Alkyl, C₁-C₃-Halogenalkyl mit 1 bis 7 Halogenatomen, Cyclopropyl, Halogencyclopropvl, C₂-C₃-Alkenyl, C₂-C₃-Alkinyl, C₁-C₃-Alkoxy, C₂-C₃-Halogenalkenyl und C₂-C₃-Halogenalkinyl mit 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe C₁-C₃-Halogenalkyl mit 1 bis 7 Halogenatomen, Cyclopropyl, Halogencyclopropyl, C₂-C₃-Alkenyl, C₂-C₃-Alkinyl, C₂-C₃-Halogenalkenyl und C₂-C₃-Halogenalkinyl mit 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyan und Nitro oder durch ein bis vier Reste aus der Gruppe C₁-C₃-Alkyl, C₁-C₃-Alkoxy und Halogen substituiertes 3-Pyridyl;
bedeuten,
dadurch gekennzeichnet, dass man eine Verbindung der Formel 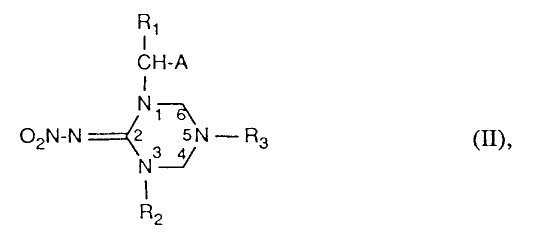 worin
- R₃: unsubstituiertes oder substituiertes C₁-C₁₀-Alkyl, C₃-C₆-Cycloalkyl, Phenyl oder Benzyl bedeutet;
hydrolysiert, gefunden.

Die erfindungsgemäss hergestellten substituierten 2-Nitroguanidine können auch als Doppelbindungsisomere bezüglich der -N=C(2)-Bindung und in ihren tautomeren Formen (Formeln I, Ia, Ib) auftreten:

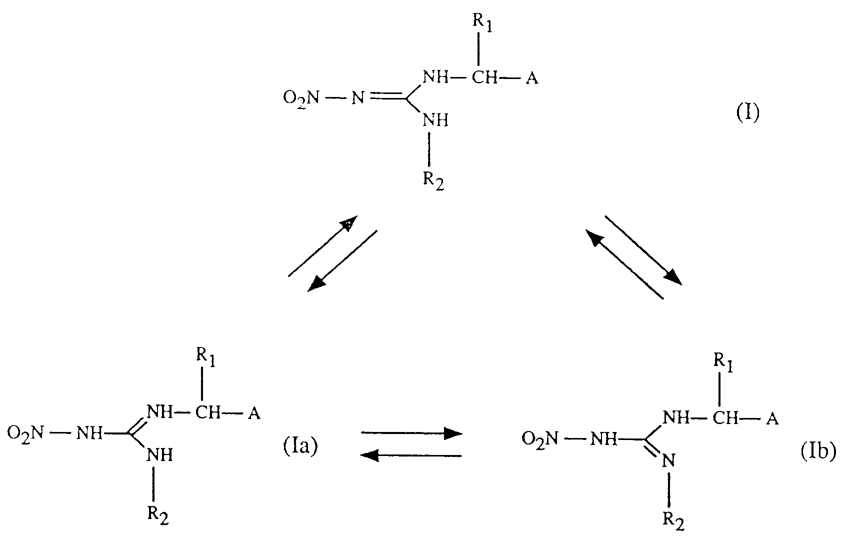

Die erfindungsgemässe Formel I ist demnach in dem Sinne zu verstehen, dass auch die entsprechenden Doppelbindungsisomeren und die Strukturen gemäss den Formeln Ia und Ib in der Schreibweise der Formel I eingeschlossen sind.

In der Definition der vorstehenden Formeln I und II sollen die einzelnen generischen Begriffe wie folgt verstanden werden:

Bei den als Substituenten in Betracht kommenden Halogenatomen handelt es sich sowohl um Fluor und Chlor als auch um Brom und Jod, wobei Fluor, Chlor und Brom bevorzugt sind. Halogen ist dabei als selbständiger Substituent oder als Teil eines Substituenten zu verstehen wie im Halogenalkyl, Halogenalkylthio, Halogenalkoxy, Halogencycloalkyl, Halogenalkenyl, Halogenalkinyl, Halogenallyloxy oder Halogenallylthio. Die als Substituenten in Betracht kommenden Alkyl-, Alkylthio-, Alkenyl-, Alkinyl- und Alkoxyreste können geradkettig oder verzweigt sein. Als Beispiele solcher Alkyle seien Methyl, Aethyl, Propyl, Isopropyl, Butyl, i-Butyl, sek.-Butyl oder tert.-Butyl genannt. Als geeignete Alkoxyreste seien unter anderem genannt: Methoxy, Aethoxy, Propoxy, Isopropoxy oder Butoxy und ihre Isomeren. Alkylthio steht beispielsweise für Methylthio, Aethylthio, Isopropylthio, Propylthio oder die isomeren Butylthio. Sind die als Substituenten in Betracht kommenden Alkyl-, Alkoxy-, Alkenyl-, Alkinyl- oder Cycloalkylgruppen durch Halogen substituiert, so können sie nur teilweise oder auch perhalogeniert sein. Dabei gelten für Halogen, Alkyl und Alkoxy die oben gegebenen Definitionen. Beispiele der Alkylelemente dieser Gruppen sind das ein- bis dreifach durch Fluor, Chlor und/oder Brom substituierte Methyl wie beispielsweise CHF₂ oder CF₃; das ein- bis fünffach durch Fluor, Chlor und/oder Brom substituierte Aethyl wie zum Beispiel CH₂CF₃, CF₂CF₃, CF₂CCl₃, CF₂CHCl₂, CF₂CHF₂, CF₂CFCl₂, CF₂CHBr₂, CF₂CHClF, CF₂CHBrF oder CClFCHClF; das ein- bis siebenfach durch Fluor, Chlor und/oder Brom substituierte Propyl oder Isopropyl wie beispielsweise CH₂CHBrCH₂Br, CF₂CHFCF₃, CH₂CF₂CF₃ oder CH(CF₃)₂; das ein- bis neunfach durch Fluor, Chlor und/oder Brom substituierte Butyl oder eines seiner Isomeren wie zum Beispiel CF(CF₃)CHFCF₃ oder CH₂(CF₂)₂CF₃; 2-Chlorcyclopropyl oder 2,2-Difluorcyclopropyl; 2,2-Difluorvinyl, 2,2-Dichlorvinyl, 2-Chloralkyl, 2,3-Dichlorvinyl oder 2,3-Dibromvinyl.

Sind die definierten Alkyl-, Alkoxy- oder Cycloalkylgruppen durch andere Substituenten substituiert, so können sie ein- oder mehrfach durch den gleichen oder verschiedene der aufgezählten Substituenten substituiert sein. Vorzugsweise sind in den substituierten Gruppen ein oder zwei weitere Substituenten vorhanden. Bei den als Substituenten in Betracht kommenden Cycloalkylresten handelt es sich beispielsweise um Cyclopropyl, Cyclobutyl, Cyclopentyl oder Cyclohexyl. Alkenyl- und Alkinylgruppen enthalten eine ungesättigte Kohlenstoff-Kohlenstoff-Bindung. Typische Vertreter sind Allyl, Methallyl oder Propargyl, aber auch Vinyl und Äthinyl. Die Doppel- oder Dreifachbindungen in Allyloxy, Propargyloxy, Allylthio oder Propargylthio sind von der Verknüpfungsstelle zum Heteroatom (O oder S) vorzugsweise durch ein gesättigtes Kohlenstoffatom getrennt.

Das erfindungsgemässe Hydrolyseverfahren wird vorzugsweise als saure Hydrolyse, besonders als saure Hydrolyse unter Normaldruck und bei einer Temperatur von 0 bis 120°C, vorzugsweise 20 bis 80°C, in einem gegenüber den Reaktionsteilnehmern inerten Lösungsmittel oder Verdünnungsmittel durchgeführt. Bevorzugt hydrolysiert man eine Verbindung der Formel II in einem wässrig-saurem Milieu, wobei als Säure Mineralsäuren, wie HCl oder H₂SO₄, und organische Säuren, wie Alkylcarbonsäuren und Sulfonsäuren, verwendet werden können. Als Lösungsmittel eignen sich in besonderer Weise Alkohole, wie Methanol, Aethanol und Propanol, sowie speziell Wasser. Weitere geeignete Lösungsmittel sind z.B. Aether, wie Tetrahydrofuran und Dioxan, sowie andere Lösungsmittel, die die Reaktion nicht beeinträchtigen. Die Lösungsmittel können auch als Gemische verwendet werden.

In Formel II können für den Rest R₃ als Substituenten der Alkyl-,C₃-C₆-Cycloalkyl-, Phenyl- oder Benzyl-Gruppen, Halogen oder eine oder mehrere, beliebige organische Reste, welche bevorzugt über ein C-, O-, N- oder S-Atom an die Alkyl-, Cycloalkyl-, Phenyl- oder Benzyl-Gruppen gebunden sind, in Frage kommen.

Zur Durchführung des erfindungsgemässen Verfahrens werden bevorzugt solche Verbindungen der Formel II als Ausgangsmaterial verwendet, worin R₃ C₁-C₁₀-Alkyl, C₃-C₆-Cycloalkyl, mit 1-12 Resten aus der Gruppe Halogen, Hydroxy, C₁-C₄-Alkoxy, C₁-C₄-Haloalkoxy mit 1 bis 9 Halogenatomen, Di-(C₁-C₄-Alkyl)-amino und C₁-C₅-Alkoxycarbonyl substituiertes C₁-C₁₀-Alkyl, mit 1-4 C₁-C₄-Alkylresten oder Halogenatomen substituiertes C₃-C₆-Cycloalkyl, Phenyl, Benzyl, oder mit 1-3 Ringsubstituenten aus der Gruppe Halogen, C₁-C₄-Alkyl, C₁-C₄-Haloalkyl mit 1 bis 9 Halogenatomen, C₁-C₄-Alkoxy, C₁-C₄-Haloalkoxy mit 1 bis 9 Halogenatomen, C₁-C₄-Alkylthio, Nitro und Cyano substituiertes Phenyl oder Benzyl, besonders C₁-C₃-Alkyl, Cyclopropyl, Cyclohexyl, Phenyl oder Benzyl bedeutet.

Die Heterocyclen A können unsubstituiert sein oder je nach Substitutionsmöglichkeiten des Ringsystems bis zu vier Substituenten tragen, wie sie unter Formel I angegeben sind. Bevorzugt tragen diese Heterocyclen ein bis drei Substituenten aus der Gruppe Halogen, C₁-C₃-Alkyl, C₁-C₃-Halogenalkyl und C₁-C₃-Halogenalkoxy mit je 1 bis 7 Halogenatomen und C₁-C₃-Alkoxy. Besonders bevorzugte Heterocyclen A sind zum Beispiel 3-Pyridyl, 2-Halogenpyrid-5-yl, 2,3-Dihalogenpyrid-5-yl, 2-Halogenthiazol-4-yl, 1-Oxido-3-pyridinio, 2-Halogen-1-oxido-5-pyridinio und 2,3-Dihalogen-1-oxido-5-pyridinio.

Weiterhin werden vorzugsweise erfindungsgemäss Verbindungen der Formel I hergestellt, worin der Rest B für einen Phenyl-, 3-Pyridyl- oder 5-Thiazolylrest, welcher unsubstituiert oder durch ein bis zwei Reste aus der Gruppe Halogen, C₁-C₃-Alkyl, C₁-C₃-Halogenalkyl sowie C₁-C₃-Halogenalkoxy mit je 1 bis 7 Halogenatomen und C₁-C₃-Alkoxy substituiert sein kann.

Unter den erfindungsgemäss herzustellenden Verbindungen der Formel I sind solche herauszuheben, in denen R₁ Wasserstoff, R₂ Wasserstoff, Methyl, Äthyl oder Cyclopropyl und A Pyridyl, 1-Oxidopyridinio, Thiazolyl oder jeweils durch ein bis drei Substituenten aus der Gruppe Halogen, C₁-C₃-Alkyl, C₁-C₃-Halogenalkyl sowie C₁-C₃-Halogenalkoxy mit 1 bis 7 Halogenatomen und C₁-C₃-Alkoxy substituiertes Pyridyl, 1-Oxidopyridinio oder Thiazolyl bedeuten. In diesem Sinne ist auch die Herstellung solcher Verbindungen der Formel I von Interesse, worin
a) R₁ für Wasserstoff steht; und/oder
b) R₂ Wasserstoff, C₁-C₃-Alkyl oder Cyclopropyl bedeutet; und/oder
c) A 2-Chlorpyrid-5-yl oder 2-Chlorthiazol-5-yl bedeutet.
Biologisch besonders interessant sind Verbindungen der Formel I, worin R₂ Methyl bedeutet

Die erfindungsgemäss hergestellten Verbindungen der Formel I sind bei günstiger Warmblüter-, Fisch- und Pflanzenverträglichkeit wertvolle Wirkstoffe in der Schädlingsbekämpfung. Insbesondere sind die Verbindungen der Formel I geeignet zur Bekämpfung von Insekten und Spinnentieren, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren vorkommen. Die Verbindungen sind vor allem wirksam gegen saugende pflanzenschädigende Insekten, insbesondere gegen Aphiden und Zikaden. Pestizid wirksame substituierte 2-Nitroguanidine vom erfindungsgemäss herstellbaren Typ werden z.B. in den EP-Patentanmeldungen 376.279,375.907 und 383.091 beschrieben.

Die für das erfindungsgemässe Verfahren in Betracht kommenden Ausgangsverbindungen der Formel II sind teilweise neuartige 2-Nitroimino-1,3,5-triazacyclohexanderivate. Sofern sie neu sind, können sie analog zu einem Verfahren hergestellt werden, welches in EP-A-386'565 beschrieben ist.

### Beispiel 1: (Herstellung von Verbindungen der Formel I)

### a) Herstellung von 1-(2-Chlorpyrid-5-ylmethyl)-2-nitro-3-n-propylguanidin:

Eine Lösung von 1,35 g 1-(2-Chlorpyrid-5-ylmethyl)-2-nitroimino-3,5-(di-n-propyl)-1,3,5-triazacyclohexan, 3 ml Essigsäure und 2 ml Wasser in 10 ml Methanol wird während 3 Tagen auf 50°C erhitzt. Anschliessend wird der Ansatz auf 100 ml Essigsäureäthylester gegossen und nacheinander mit je 50 ml gesättigter NaCl-, gesättigter NaHCO₃- und gesättigter NaCl-Lösung gewaschen. Die abgetrennte organische Phase wird über MgSO₄ getrocknet und eingedampft. Das als Rückstand erhaltene Rohprodukt wird an Kieselgel mit Hexan/Essigsäureäthylester (1:2) chromatographiert. Man erhält so 0,80 g der Titelverbindung, Smp. 122-123°C, der Formel

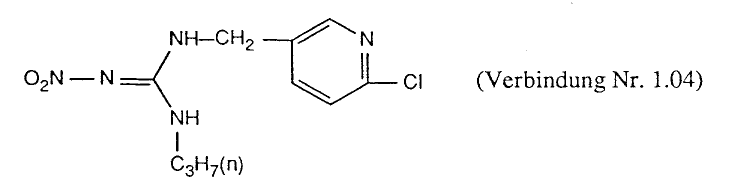

### b) Herstellung von 1-(2-Chlorpyrid-5-ylmethyl)-2-nitro-3-methylguanidin:

Eine Lösung von 4,25 g 1-(2-Chlorpyrid-5-ylmethyl)-2-nitroimino-3-methyl-5-n-propyl-1,3,5-triazacyclohexan in 26 ml Methanol wird mit 26 ml 1N HCl versetzt und während 16 Stunden bei Raumtemperatur gerührt. Das Reaktionsgemisch wird filtriert, die abfiltrierten Kristalle werden mit wenig Methanol nachgewaschen und getrocknet. Man erhält so 2,51 g der Titelverbindung, Smp. 148-150°C, der Formel 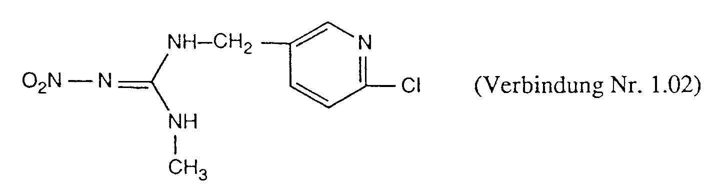 Analog der obigen Arbeitsweisen können auch die folgenden in Tabelle I aufgeführten Verbindungen der Formel I erhalten werden:

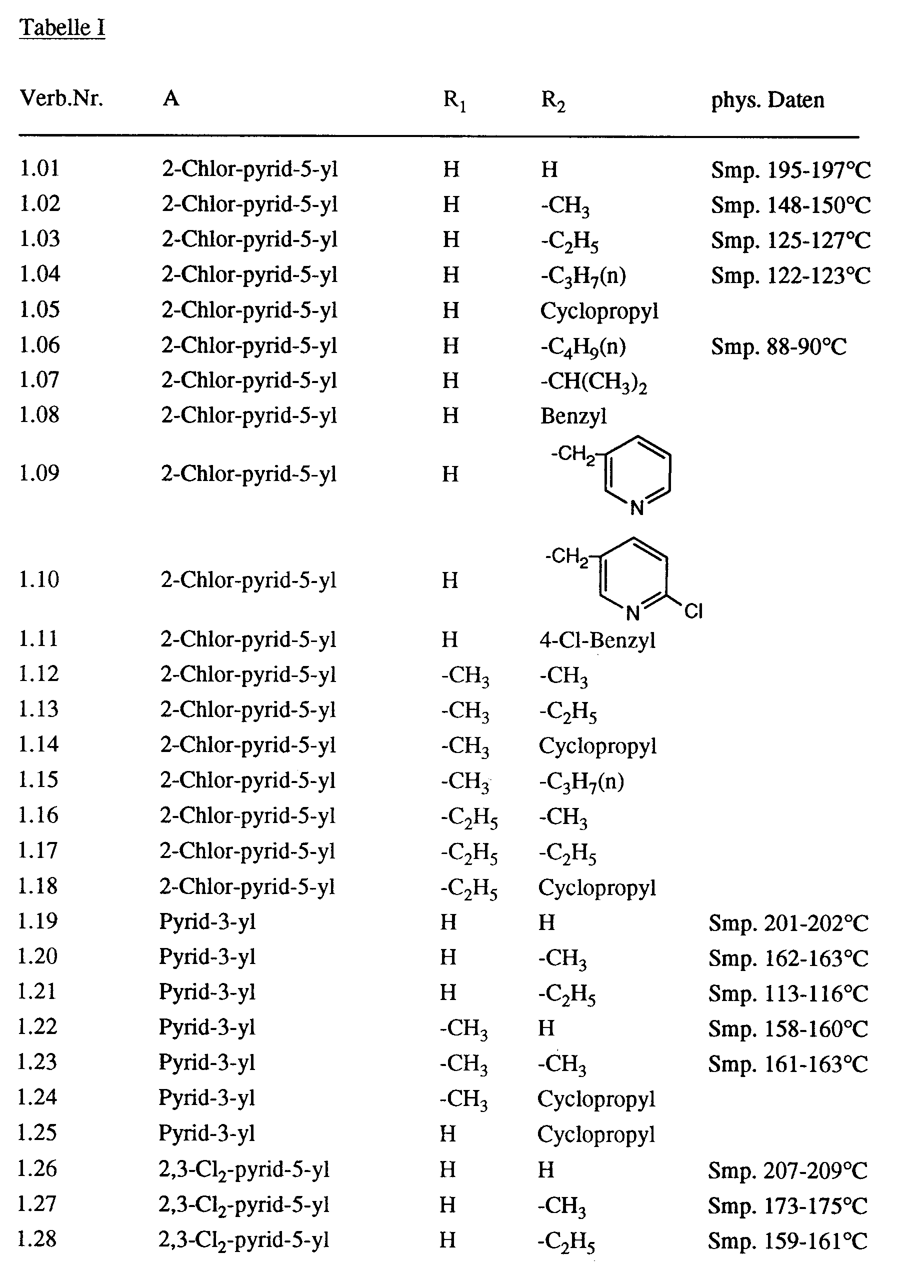

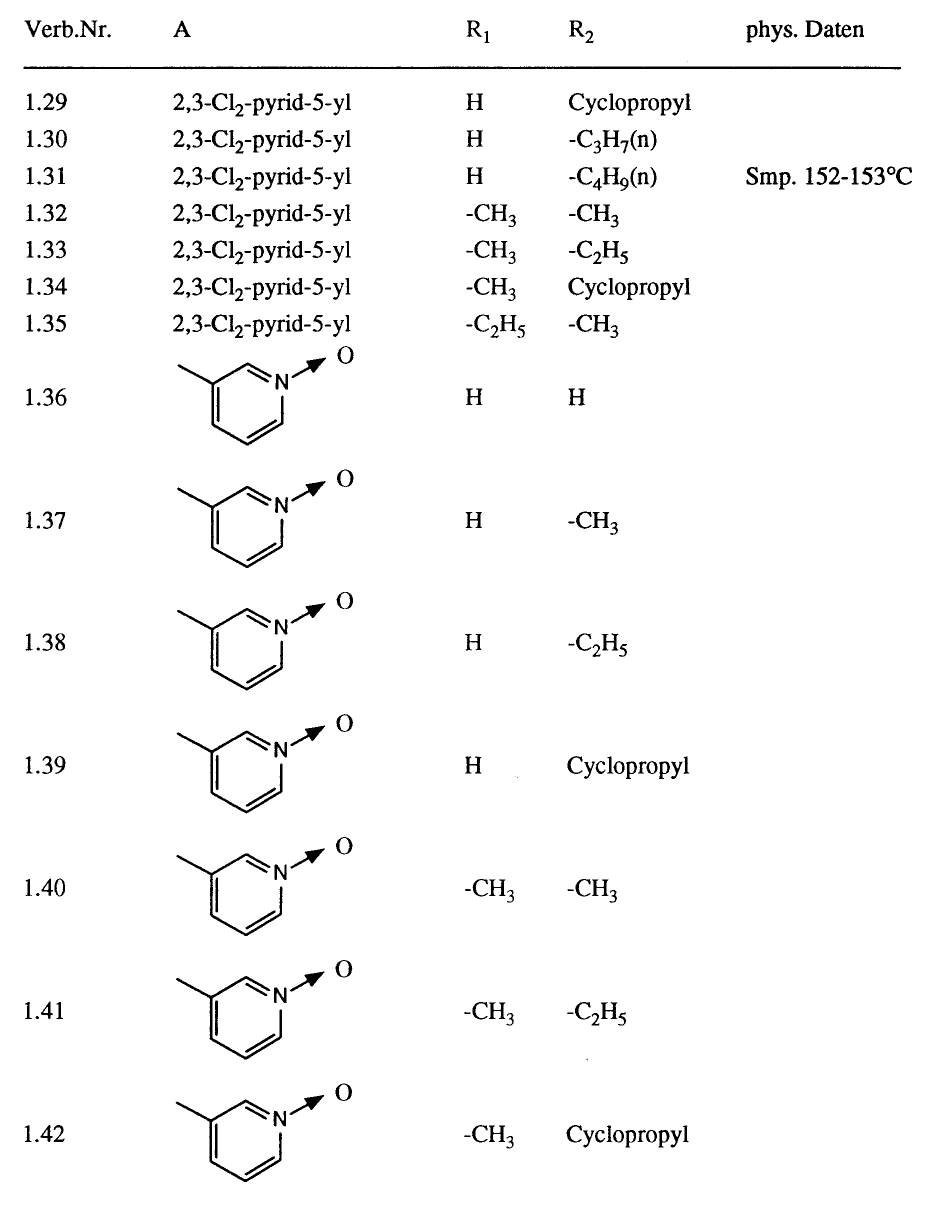

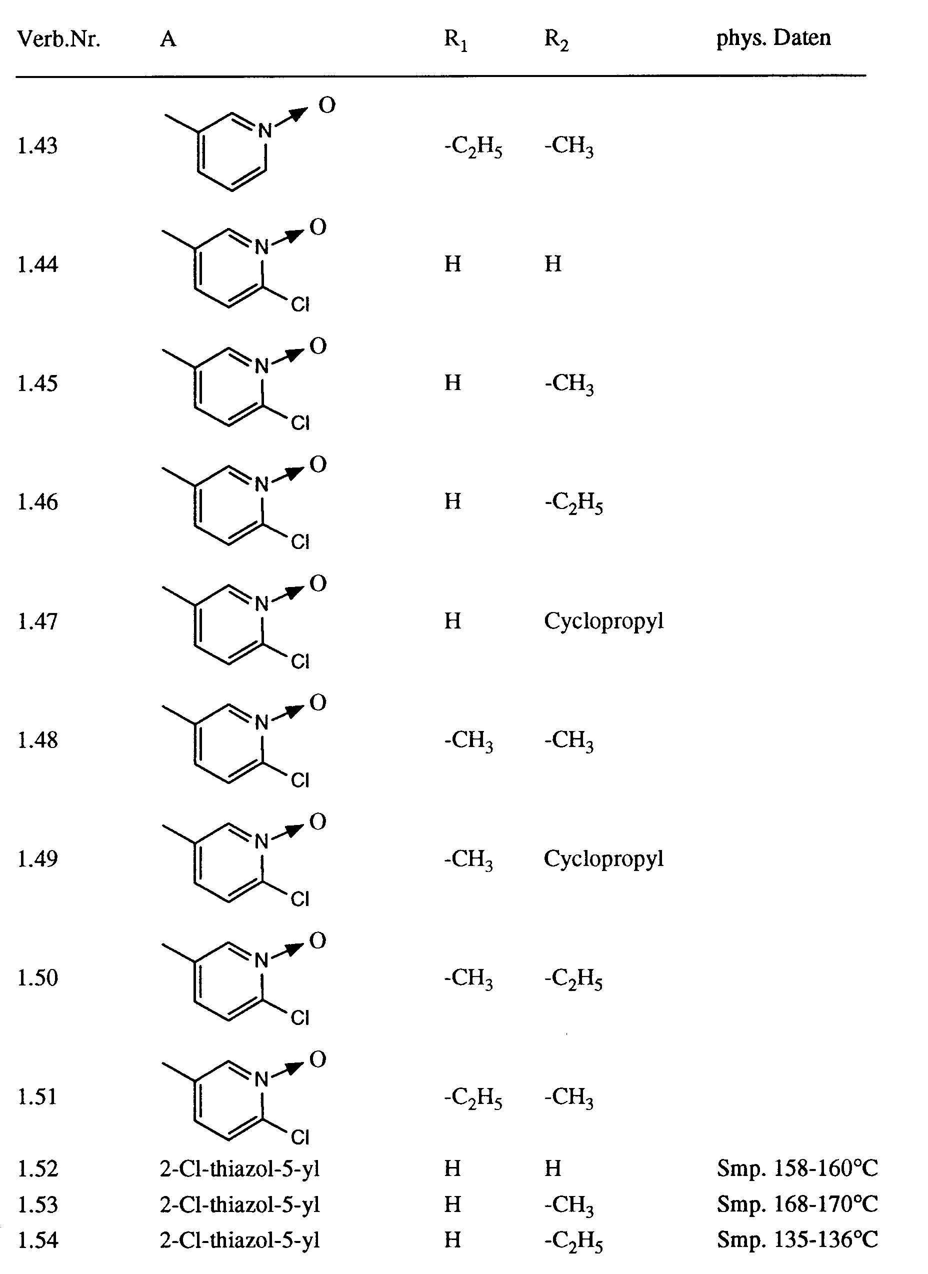

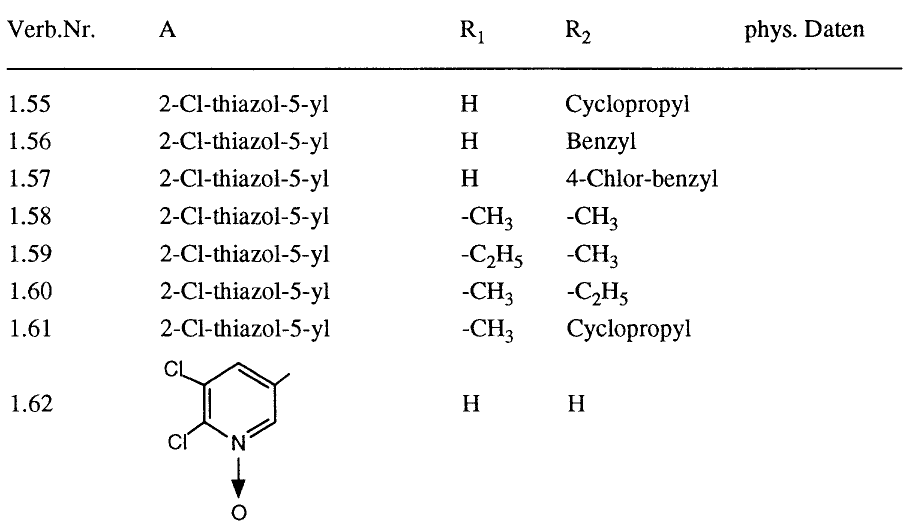

## Patentansprüche

1. Verfahren zur Herstellung einer Verbindung der Formel 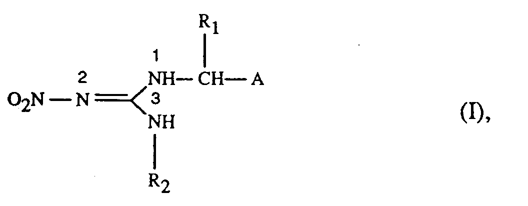 worin
R₁ Wasserstoff oder C₁-C₄-Alkyl,
R₂ Wasserstoff, C₁-C₆-Alkyl, C₃-C₆-Cycloalkyl oder einen Rest -CH₂B;
A einen unsubstituierten oder, je nach Substitutionsmöglichkeiten des Ringsystems, ein- bis vierfach substituierten Pyridyl-, 1-Oxidopyridinio- oder Thiazolyl-Rest, der ein bis zwei Substituenten aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃-Halogenalkenyl und C₂-C₃-Halogenalkinyl mit 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyan und Nitro und ein bis vier Substituenten aus der Gruppe C₁-C₃-Alkyl, C₁-C₃-Alkoxy und Halogen enthalten kann; und
B Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, durch ein bis zwei Substituenten aus der Gruppe C₁-C₃-Alkyl, C₁-C₃-Halogenalkyl mit 1 bis 7 Halogenatomen, Cyclopropyl, Halogencyclopropyl, C₂-C₃-Alkenyl, C₂-C₃-Alkinyl, C₁-C₃-Alkoxy, C₂-C₃-Halogenalkenyl und C₂-C₃-Halogenalkinyl mit 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe C₁-C₃-Halogenalkyl mit 1 bis 7 Halogenatomen, Cyclopropyl, Halogencyclopropyl, C₂-C₃-Alkenyl, C₂-C₃-Alkinyl, C₂-C₃-Halogenalkenyl und C₂-C₃-Halogenalkinyl mit 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyan und Nitro oder durch ein bis vier Reste aus der Gruppe C₁-C₃-Alkyl, C₁-C₃-Alkoxy und Halogen substituiertes 3-Pyridyl;
bedeuten,
dadurch gekennzeichnet, dass man eine Verbindung der Formel 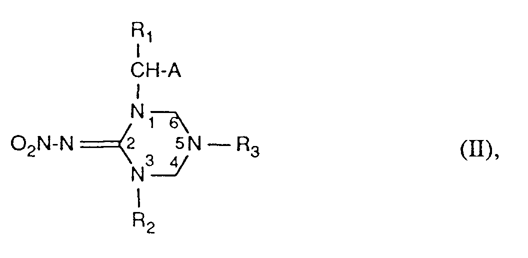 worin
R₃ unsubstituiertes oder substituiertes C₁-C₁₀-Alkyl, C₃-C₆-Cycloalkyl, Phenyl oder Benzyl bedeutet;
hydrolysiert.

2. Verfahren gemäss Anspruch 1, dadurch gekennzeichnet, dass man die Verbindung der Formel II sauer hydrolisiert.

3. Verfahren gemäss Anspruch 1 oder 2, dadurch gekennzeichnet, dass man eine Verbindung der Formel II verwendet,
worin
R₃ C₁-C₁₀-Alkyl, C₃-C₆-Cycloalkyl, mit 1-12 Resten aus der Gruppe Halogen, Hydroxy, C₁-C₄-Alkoxy, C₁-C₄-Haloalkoxy mit 1 bis 9 Halogenatomen, Di-(C₁-C₄-Alkyl)amino und C₁-C₅-Alkoxycarbonyl substituiertes C₁-C₁₀-Alkyl, mit 1-4 C₁-C₄-Alkylresten oder Halogenatomen substituiertes C₃-C₆-Cycloalkyl, Phenyl, Benzyl, oder mit 1-3 Ringsubstituenten aus der Gruppe Halogen, C₁-C₄-Alkyl, C₁-C₄-Haloalkyl mit 1 bis 9 Halogenatomen, C₁-C₄-Alkoxy, C₁-C₄-Haloalkoxy mit 1 bis 9 Halogenatomen, C₁-C₄-Alkylthio, Nitro und Cyano substituiertes Phenyl oder Benzyl bedeutet.

4. Verfahren gemäss einem der Ansprüche 1 bis 3, dadurch gekennzeichnet dass eine Verbindung der Formel I hergestellt wird, worin der Rest A unsubstituiert ist oder, je nach Substitutionsmöglichkeiten des Ringsystems, ein bis drei Substituenten aus der Gruppe Halogen, C₁-C₃-Alkyl, C₁-C₃-Halogenalkyl sowie C₁-C₃-Halogenalkoxy mit 1 bis 7 Halogenatomen und C₁-C₃-Alkoxy trägt.

5. Verfahren gemäss einem der Ansprüche 1 bis 4, dadurch gekennzeichnet, dass eine Verbindung der Formel I hergestellt wird, worin der Rest B für einen Phenyl-, 3-Pyridyl- oder 5-Thiazolylrest steht, welcher unsubstituiert oder durch ein bis zwei Reste aus der Gruppe Halogen, C₁-C₃-Alkyl, C₁-C₃-Halogenalkyl und C₁-C₃-Halogenalkoxy mit 1 bis 7 Halogenatomen und C₁-C₃-Alkoxy substituiert ist.

6. Verfahren gemäss einem der Ansprüche 4 oder 5, dadurch gekennzeichnet, dass eine Verbindung der Formel I hergestellt wird, worin der Rest A für 3-Pyridyl, 2-Halogen-pyrid-5-yl, 2,3-Dihalogenpyrid-5-yl oder 2-Halogenthiazol-5-yl, 1-Oxido-3-pyridinio, 2-Halogen-1-oxido-5-pyridinio oder 2,3-Dihalogen- 1-oxido-5-pyridinio steht.

7. Verfahren gemäss einem der Ansprüche 1 bis 4, dadurch gekennzeichnet, dass eine Verbindung der Formel I hergestellt wird, worin R₁ Wasserstoff; R₂ Wasserstoff, Methyl, Äthyl oder Cyclopropyl und A Pyridyl, 1-Oxidopyridinio, Thiazolyl oder jeweils, je nach Substitutionsmöglichkeiten des Ringsystems, durch ein bis drei Substituenten aus der Gruppe Halogen, C₁-C₃-Alkyl, C₁-C₃-Halogenalkyl und C₁-C₃-Halogenalkoxy mit 1 bis 7 Halogenatomen und C₁-C₃Alkoxy substituiertes Pyridyl, 1-Oxidopyridinio oder Thiazolyl bedeuten.

8. Verfahren gemäss einem der Ansprüche 1 bis 7, gekennzeichnet, dass eine Verbindung der Formel I hergestellt wird, worin R₁ Wasserstoff bedeutet.

9. Verfahren gemäss einem der Ansprüch 1 bis 4,6 und 8, dadurch gekennzeichnet, dass eine Verbindung der Formel I hergestellt wird, worin R₂ Wasserstoff, C₁-C₃-Alkyl oder Cyclopropyl bedeutet.

10. Verfahren gemäss einem der Ansprüche 1 oder 9, dadurch gekennzeichnet, dass eine Verbindung der Formel I hergestellt wird, worin A 2-Chlorpyrid-5-yl oder 2-Chlortriazol-5-yl bedeutet.

11. Verfahren gemäss einem der Ansprüche 1 bis 4 und 6 bis 10, dadurch gekennzeichnet, dass eine Verbindung der Formel I hergestellt wird, worin R₂ Methyl bedeutet.

12. Verfahren gemäss einem der Ansprüche 3 bis 11, dadurch gekennzeichnet, dass man eine Verbindung der Formel II verwendet, worin R₃ C₁-C₃-Alkyl, Cyclopropyl, Cyclohexyl, Phenyl oder Benzyl bedeutet.

## Claims

1. A process for the preparation of a compound of the formula 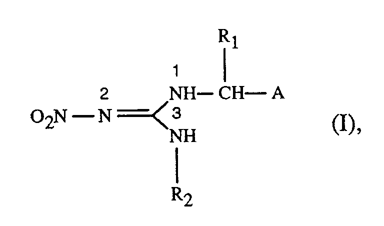 in which R₁ is hydrogen or C₁-C₄alkyl, R₂ is hydrogen, C₁-C₆alkyl, C₃-C₆cycloalkyl or a radical -CH₂B; A is an unsubstituted or, depending on the substitution possibilities of the ring system, a mono- to tetrasubstituted pyridyl, 1-oxidopyridinio or thiazolyl radical, which may contain one or two substituents from the group comprising C₁-C₃haloalkyl having 1 to 7 halogen atoms, cyclopropyl, halocyclopropyl having 1 to 3 halogen atoms, C₂-C₃alkenyl, C₂-C₃alkynyl, C₂-C₃haloalkenyl and C₂-C₃haloalkynyl having 1 to 4 halogen atoms, C₁-C₃haloalkoxy having 1 to 7 halogen atoms, C₁-C₃alkylthio, C₁-C₃haloalkylthio having 1 to 7 halogen atoms, allyloxy, propargyloxy, allylthio, propargylthio, haloallyloxy, haloallylthio, cyano and nitro and one to four substituents from the group comprising C₁-C₃alkyl, C₁-C₃alkoxy and halogen; and B is phenyl, cyanophenyl, nitrophenyl, halophenyl having 1 to 3 halogen atoms, phenyl substituted by C₁-C₃alkyl, C₁-C₃haloalkyl having 1 to 7 halogen atoms, C₁-C₃alkoxy or C₁-C₃haloalkoxy having 1 to 7 halogen atoms, 3-pyridyl, 5-thiazolyl, 5-thiazolyl substituted by one or two substituents from the group comprising C₁-C₃alkyl, C₁-C₃haloalkyl having 1 to 7 halogen atoms, cyclopropyl, halocyclopropyl, C₂-C₃alkenyl, C₂-C₃alkynyl, C₁-C₃alkoxy, C₂-C₃haloalkenyl and C₂-C₃haloalkynyl having 1 to 4 halogen atoms, C₁-C₃haloalkoxy having 1 to 7 halogen atoms, C₁-C₃alkylthio, C₁-C₃haloalkylthio having 1 to 7 halogen atoms, allyloxy, propargyloxy, allylthio, propargylthio, haloallyloxy, haloallylthio, halogen, cyano and nitro, or pyridyl substituted by one or two radicals from the group comprising C₁-C₃haloalkyl having 1 to 7 halogen atoms, cyclopropyl, halocyclopropyl, C₂-C₃alkenyl, C₂-C₃alkynyl, C₂-C₃haloalkenyl and C₂-C₃haloalkynyl having 1 to 4 halogen atoms, C₁-C₃haloalkoxy having 1 to 7 halogen atoms, C₁-C₃alkylthio, C₁-C₃haloalkylthio having 1 to 7 halogen atoms, allyloxy, propargyloxy, allylthio, propargylthio, haloallyloxy, haloallylthio, cyano and nitro or by one to four radicals from the group comprising C₁-C₃alkyl, C₁-C₃alkoxy and halogen,
which comprises hydrolysing a compound of the formula 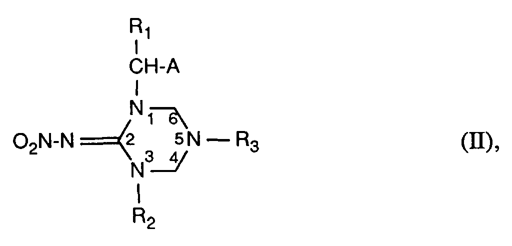 in which R₃ is unsubstituted or substituted C₁-C₁₀alkyl, C₃-C₆cycloalkyl, phenyl or benzyl.

2. A process according to claim 1, wherein the compound of the formula II is hydrolysed by acid.

3. A process according to claim 1 or 2, wherein a compound of the formula II is used in which R₃ is C₁-C₁₀alkyl, C₃-C₆cycloalkyl, C₁-C₁₀allyl substituted by 1-12 radicals from the group comprising halogen, hydroxyl, C₁-C₄alkoxy, C₁-C₄haloalkoxy having 1 to 9 halogen atoms, di-(C₁-C₄alkyl)amino and C₁-C₅alkoxycarbonyl, C₃-C₆cycloalkyl substituted by 1-4 alkyl radicals or halogen atoms, phenyl, benzyl, or phenyl or benzyl substituted by 1-3 ring substituents from the group comprising halogen, C₁-C₄alkyl, C₁-C₄haloalkyl having 1 to 9 halogen atoms, C₁-C₄alkoxy, C₁-C₄haloalkoxy having 1 to 9 halogen atoms, C₁-C₄alkylthio, nitro and cyano.

4. A process according to any one of claims 1 to 3, wherein a compound of the formula I is prepared in which the radical A is unsubstituted or, depending on the substitution possibilities of the ring system, carries one to three substituents from the group comprising halogen, C₁-C₃alkyl, C₁-C₃haloalkyl and C₁-C₃haloalkoxy having 1 to 7 halogen atoms, and C₁-C₃alkoxy.

5. A process according to any one of claims 1 to 4, wherein a compound of the formula I is prepared in which the radical B is a phenyl, pyridyl or thiazolyl radical, which is unsubstituted or substituted by one or two radicals from the group comprising halogen, C₁-C₃alkyl, C₁-C₃haloalkyl and C₁-C₃haloalkoxy having 1 to 7 halogen atoms, and C₁-C₃alkoxy.

6. A process according to either of claims 4 or 5, wherein a compound of the formula I is prepared in which the radical A is 3-pyridyl, 2-halopyrid-5-yl, 2,3-dihalopyrid-5-yl or 2-halothiazol-5-yl, 1-oxido-3-pyridinio, 2-halo-1-oxido-5-pyridinio or 2,3-dihalo-1-oxido-5-pyridinio.

7. A process according to any one of claims 1 to 4, wherein a compound of the formula I is prepared in which R₁ is hydrogen; R₂ is hydrogen, methyl, ethyl or cyclopropyl and A is pyridyl, 1-oxidopyridinio, thiazolyl or pyridyl, 1-oxidopyridinio or thiazolyl in each case substituted, depending on the substitution possibilities of the ring system, by one to three substituents from the group comprising halogen, C₁-C₃alkyl, C₁-C₃haloalkyl and C₁-C₃haloalkoxy having 1 to 7 halogen atoms, and C₁-C₃alkoxy.

8. A process according to any one of claims 1 to 7, wherein a compound of the formula I is prepared in which R₁ is hydrogen.

9. A process according to any one of claims 1 to 4,6 and 8, wherein a compound of the formula I is prepared in which R₂ is hydrogen, C₁-C₃alkyl or cyclopropyl.

10. A process according to either of claims 1 or 9, wherein a compound of the formula I is prepared in which A is 2-chloropyrid-5-yl or 2-chlorotriazol-5-yl.

11. A process according to any one of claims 1 to 4 and 6 to 10, wherein a compound of the formula I is prepared in which R₂ is methyl.

12. A process according to any one of claims 3 to 11, wherein a compound of the formula II is used in which R₃ is C₁-C₃alkyl, cyclopropyl, cyclohexyl, phenyl or benzyl.

## Revendications

1. Procédé pour la préparation d'un composé de formule 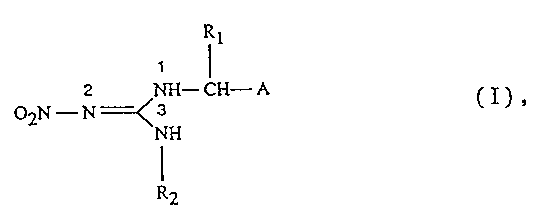 où
R₁ représente l'hydrogène ou un alkyle en C₁-C₄;
R₂ représente l'hydrogène, un alkyle en C₁-C₆, un cycloalkyle en C₃-C₆ ou un reste -CH₂B;
A représente les restes pyridyle, 1-oxydopyridinio ou thiazolyle non substitués ou substitués une à quatre fois, suivant, les `possibilités de substitution de la combinaison cyclique, ces restes pouvant contenir un ou deux substituants provenant du groupe constitué par les halogénoalkyles en C₁-C₃ comportant 1 à 7 atomes d'halogène, le cyclopropyle, les halogénocyclopropyles comportant 1 à 3 atomes d'halogène, les alcényles en C₂-C₃, les alcynyles en C₂-C₃, les halogénoalcényles en C₂-C₃ et les halogénoalcynyles en C₂-C₃ comportant 1 à 4 atomes d'halogène, les halogénoalcoxy en C₁-C₃ comportant 1 à 7 atomes d'halogène, les alkyles en C₁-C₃ thio, les halogénoalkyles en C₁-C₃ thio comportant 1 à 7 atomes d'halogène, l'allyloxy, le propargyloxy, l'allylthio, le propargylthio, les halogénoallyloxy, les halogénoallylthio, le cyano et le nitro et un à quatre substituants provenant du groupe constitué par les alkyles en C₁-C₃, les alcoxy en C₁-C₃ et les halogènes; et
B représente le phényle, le cyanophényle, le nitrophényle, un halogénophényle comportant 1 à 3 atomes d'halogène, un phényle substitué par un alkyle en C₁-C₃, un halogénoalkyle en C₁-C₃ comportant 1 à 7 atomes d'halogène, un alcoxy en C₁-C₃ ou un halogénoalcoxy en C₁-C₃ comportant 1 à 7 atomes d'halogène, le 3-pyridyle, le 5-thiazolyle, un 5-thiazolyle substitué par un ou deux substituants provenant du groupe constitué par les alkyles en C₁-C₃, les halogénoalkyles en C₁-C₃ comportant 1 à 7 atomes d'halogène, le cyclopropyle, les halogénocyclopropyles, les alcényles en C₂-C₃, les alcynyles en C₂-C₃, les alcoxy en C₁-C₃, les halogénoalcényles en C₂-C₃ et les halogénoalcynyles en C₂-C₃ comportant 1 à 4 atomes d'halogène, les halogénoalcoxy en C₁-C₃ comportant 1 à 7 atomes d'halogène, les alkyles en C₁-C₃ thio, les halogénoalkyles en C₁-C₃ thio comportant 1 à 7 atomes d'halogène, l'allyloxy, le propargyloxy, l'allylthio, le propargylthio, les halogénoallyloxy, les halogénoallylthio, les halogènes, le cyano et le nitro; ou un 3-pyridyle substitué par un ou deux restes provenant du groupe constitué par les halogénoalkyles en C₁-C₃ comportant 1 à 7 atomes d'halogène, le cyclopropyle, les halogénocyclopropyle, les alcényles en C₂-C₃, les alcynyles en C₂-C₃, les halogénoalcényles en C₂-C₃ et les halogénoalcynyles en C₂-C₃ comportant 1 à 4 atomes d'halogène, les halogénoalcoxy en C₁-C₃ comportant 1 à 7 atomes d'halogène, les alkyles en C₁-C₃ thio, les halogénoalkyles en C₁-C₃ thio comportant 1 à 7 atomes d'halogène, l'allyloxy, le propargyloxy, l'allylthio, le propargylthio, les halogénoallyloxy, les halogénoallylthio, le cyano et le nitro ou un 3-pyridyle substitué par un à quatre restes provenant du groupe constitué par les alkyles en C₁-C₃, les alcoxy en C₁-C₃ et les halogènes;
caractérisé en ce que l'on hydrolyse un composé de formule 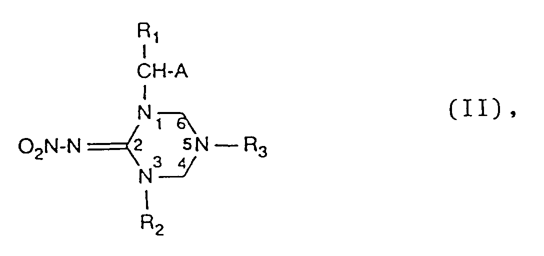 où
R₃ représente un alkyle en C₁-C₁₀, un cycloalkyle en C₃-C₆, le phényle ou le benzyle, substitués ou non.

2. Procédé selon la revendication 1, caractérisé en ce que l'on soumet le composé de formule II à une hydrolyse acide.

3. Procédé selon la revendication 1 ou 2, caractérisé en ce que' l'on utilise un composé de formule II où R₃ représente un alkyle en C₁-C₁₀, un cycloalkyle en C₃-C₆, un alkyle en C₁-C₁₀ substitué par 1 à 12 restes provenant du groupe constitué par les halogènes, l'hydroxy, les alcoxy en C₁-C₄, les haloalcoxy en C₁-C₄ comportant 1 à 9 atomes d'halogène, les di-(alkyles en C₁-C₄)amino et les alcoxy en C₁-C₅ carbonyle, les cycloalkyles en C₃-C₆ substitués par 1 à 4 restes alkyles en C₁-C₄ ou des atomes d'halogène, le phényle, le benzyle ou un phényle ou un benzyle substitué par 1 à 3 substituants sur le noyau provenant du groupe constitué par les halogènes, les alkyles en C₁-C₄, les haloalkyles en C₁-C₄ comportant 1 à 9 atomes d'halogène, les alcoxy en C₁-C₄, les haloalcoxy en C₁-C₄ comportant 1 à 9 atomes d'halogène, les alkyles en C₁-C₄ thio, le nitro et le cyano.

4. Procédé selon l'une des revendications 1 à 3, caractérisé en ce que l'on prépare un composé de formule I où le reste A est non substitué ou porte, suivant les possibilités de substitution de la combinaison cyclique, un à trois substituants provenant du groupe constitué par les halogènes, les alkyles en C₁-C₃, les halogénoalkyles en C₁-C₃ et les halogénoalcoxy en C₁-C₃ comportant. 1 à 7 atomes d'halogène et les alcoxy en C₁-C₃.

5. Procédé selon l'une des revendications 1 à 4, caractérisé en ce que l'on prépare un composé de formule I où le reste B représente les restes phényle, 3-pyridyle ou 5-thiazolyle, ces restes étant non substitués ou substitués par un ou deux restes provenant du groupe constitué par les halogènes, les alkyles en C₁-C₃, les halogénoalkyles en C₁-C₃ et les halogénoalcoxy en C₁-C₃ comportant 1 à 7 atomes d'halogène et les alcoxy en C₁-C₃.

6. Procédé selon l'une des revendications 4 et 5, caractérisé en ce que l'on prépare un composé de formule I où le reste A représente le 3-pyridyle, un 2-halogénopyrid-5-yle, un 2,3-dihalogénopyrid-5-yle ou un 2-halogénothiazol-5-yle, le 1-oxydo-3-pyridinio, un 2-halogéno-1-oxydo-5-pyridinio ou un 2,3-dihalogéno-1-oxydo-5-pyridinio.

7. Procédé selon l'une des revendications 1 à 4, caractérisé en ce que l'on prépare un composé de formule I où R₁ représente l'hydrogène; R₂ l'hydrogène, le méthyle, l'éthyle ou le cyclopropyle et A les pyridyle, 1-oxydopyridinio, thiazolyle ou les pyridyle, 1-oxydopyridinio ou thiazolyle substitués, suivant les possibilités de substitution de la combinaison cyclique, par un ou trois substituants provenant du groupe constitué par les halogènes, les alkyles en C₁-C_{3,} les halogénoalkyles en C₁-C₃ et les halogénoalcoxy en C₁-C₃ comportant 1 à 7 atomes d'halogène et les alcoxy en C₁-C₃.

8. Procédé selon l'une des revendications 1 à 7, caractérisé en ce que l'on prépare un composé de formule I où R₁ représente l'hydrogène.

9. Procédé selon l'une des revendications 1 à 4, caractérisé en ce que l'on prépare un composé de formule I où R₂ représente l'hydrogène, un alkyle en C₁-C₃ ou le cyclopropyle.

10. Procédé selon l'une des revendications 1 et 9, caractérisé en ce que l'on prépare un composé de formule I où A représente le 2-chloropyrid-5-yle ou le 2-chlorotriazol-5-yle.

11. Procédé selon l'une des revendications 1 à 4 et 6 à 10, caractérisé en ce que l'on prépare un composé de formule I où R₂ représente le méthyle.

12. Procédé selon l'une des revendications 3 à 11, caractérisé en ce que l'on prépare un composé de formule II où R₃ représente un alkyle en C₁-C₃, le cyclopropyle, le cyclohexyle, le phényle ou le benzyle.
